# EUROPEAN PATENT SPECIFICATION

(11) **EP 1 905 286 B1**
(45) Date of publication and mention of the grant of the patent: **14.10.2015**
(21) Application number: 06762497.3
(22) Date of filing: 07.07.2006
(51) Int. Cl.: H05H 1/34

(54) **PLASMA-GENERATING DEVICE AND PLASMA SURGICAL DEVICE**
PLASMAERZEUGUNGSEINRICHTUNG UND CHIRURGISCHE PLASMAEINRICHTUNG
DISPOSITIF GENERATEUR DE PLASMA ET DISPOSITIF CHIRURGICAL AU PLASMA

(30) Priority: 08.07.2005 SE 0501604
(43) Date of publication of application: 02.04.2008
(73) Proprietor: Plasma Surgical AB, 431 35 Mölndal (SE); Plasma Surgical Investments Limited, Tortola (VG)
(72) Inventor: SUSLOV, Nikolay, S-421 67 Västra Frölunda (SE)
(74) Representative: Somlo, Tommy
(86) International application number: PCT/EP2006/006690
(87) International publication number: WO 2007/006518

(56) References cited:
- WO-A-02/30308
- WO-A-2004/105450
- WO-A1-2004/028221
- GB-A- 1 268 843
- US-A- 5 640 843

## Description

### CLAIM OF PRIORITY

This application claims priority of a Swedish Patent Application No. 0501604-3 filed on July 8, 2005.

### FIELD OF THE INVENTION

The present invention relates to a plasma-generating device, comprising an anode, a cathode and a plasma channel which in its longitudinal direction extends at least partly between said cathode and said anode. The invention also relates to a plasma surgical device and use of a plasma surgical device in the field of surgery.

### BACKGROUND ART

Plasma devices relate to the devices which are arranged to generate a gas plasma. Such gas plasma can be used, for instance, in surgery for the purpose of causing destruction (dissection) and/or coagulation of biological tissues.

As a rule, such plasma devices are formed with a long and narrow end or the like which can easily be applied to a desired area that is to be treated, such as bleeding tissue. At the tip of the device, a gas plasma is present, the high temperature of which allows treatment of the tissue adjacent to the tip.

WO 2004/030551 (Suslov) discloses a plasma surgical device according to prior art. This device comprises a plasma-generating system with an anode, a cathode and a gas supply channel for supplying gas to the plasma-generating system. Moreover the plasma-generating system comprises a number of electrodes which are arranged between said cathode and anode. A housing of an electrically conductive material which is connected to the anode encloses the plasma-generating system and forms the gas supply channel. WO2004/105450 (Bijker et al.) is another prior art document. It discloses a cascade source provided with a cathode, a cathode housing, a plasma channel and an anode plate provided with an outflow opening connecting to the plasma channel. The cathode has a tip which is located near the bottom side of the cathode housing.

Owing to the recent developments in surgical technology, that referred to as laparoscopic (keyhole) surgery is being used more often. This implies, for example, a greater need for devices with small dimensions to allow accessibility without extensive surgery. Small instruments are also advantageous in surgical operation to achieve good accuracy.

When making plasma devices with small dimensions, there is often a risk that material adjacent to the cathode is heated to high temperatures due to the temperature of the cathode, which in some cases may exceed 3000 °C. At these temperatures there is a risk that material adjacent to the cathode is degraded and contaminates the gas plasma. Contaminated gas plasma may, for instance, introduce undesirable particles into the surgical area and may be injurious to a patient who is being treated.

Thus, there is a need for improved plasma devices, in particular plasma devices with small dimensions which can produce a high temperature plasma.

### SUMMARY OF THE INVENTION

An object of the present invention is to provide an improved plasma-generating device according to the preamble to claim 1.

Additional objects of the invention is to provide a plasma surgical device and use of such a plasma surgical device in the field of surgery.

According to one aspect of the invention, a plasma-generating device according to claim 1 is provided. The plasma chamber suitably has a cross-section which exceeds a cross-section of a plasma channel opening closest to the cathode.

By plasma channel is meant an elongate channel which is in fluid communication with the plasma chamber. The plasma channel is positioned beyond, in the direction from the cathode to the anode, the plasma chamber and extends away from the cathode towards the anode. In one embodiment, the plasma channel extends from the plasma chamber towards and through the anode. The plasma channel suitably has an outlet in the anode, through which outlet generated plasma, in operation of the device, can be discharged. A transition portion can be arranged between the plasma channel and the plasma chamber. Alternatively, the plasma chamber and the plasma channel can be in direct contact with each other.

By plasma chamber is meant an area in which a plasma-generating gas which is supplied to the plasma-generating device is mainly converted to plasma. With a device according to the invention, completely new conditions of generating such a plasma are provided.

In prior art plasma-generating devices, damage and degeneration of material due to the high temperature of the cathode are often prevented by materials adjacent to the cathode being placed at a considerably great distance from the cathode. Moreover, the cathode is often placed in direct contact with the plasma channel to prevent the occurrence of an incorrectly generated electric arc, in which case the plasma channel, due to high temperatures of the cathode, is usually given considerably large dimensions relative to the dimensions of the cathode so as not to be damaged by the high temperature in operation. Such prior art devices will thus often be given outer dimensions which typically are greater than 10 mm, which can be unwieldy and difficult to handle in applications, for instance, in what is referred to as laparoscopic surgery (keyhole surgery) and other space-limited applications.

By a plasma chamber which at least partly between the cathode end directed to the anode and the opening of the plasma channel closest to the cathode, it is possible to provide a plasma-generating device with smaller outer dimensions than those of prior art devices.

For this type of plasma-generating devices, it is not uncommon for the cathode tip in operation to have a temperature which is higher than 2500°C, in some cases higher than 3000°C.

By using a plasma chamber, a possibility of forming a space around the cathode, especially the tip of the cathode closest to the anode, can advantageously be provided. Consequently, the plasma chamber allows the outer dimensions of the plasma-generating device to be relatively small. The space around the cathode tip is convenient to reduce the risk that the high temperature of the cathode in operation damages and/or degrades material of the device, which material adjoins the cathode. In particular this is important for devices intended for surgical applications where there is a risk that degraded material can contaminate the plasma and accompany the plasma into a surgical area, which may cause detrimental effects to a patient. A plasma chamber according to the invention is particularly advantageous with long continuous times of operation.

A further advantage achieved by arranging a plasma chamber is that an electric arc which is intended to be generated between the cathode and the anode can be safely obtained since the plasma chamber allows the tip of the cathode to be positioned in the vicinity of the plasma channel opening closest to the cathode without adjoining material being damaged and/or degraded due to the high temperature of the cathode. If the tip of the cathode is positioned at too great a distance from the opening of the plasma channel, an electric arc between the cathode and adjoining structures is often generated in an unfavourable manner, thus causing incorrect operation of the device and, in some cases, also damage to the device.

A plasma-generating device according to the invention can be particularly suitable when it is desirable to provide plasma-generating devices having small outer dimensions, such as an outer diameter below 10 mm, and especially below 5 mm. Moreover, the invention is suitable to provide a plasma-generating device which can generate a plasma which often has a temperature higher than 10,000°C as the plasma is being discharged through the outlet of the plasma channel at the end of the device. For instance, the plasma discharged through the outlet of the plasma channel can have a temperature between 10,000 and 15,000 °C. Such high temperatures will be possible, for instance, through the option of making the cross-section of the plasma channel smaller when using a plasma chamber according to the invention. Smaller dimensions of the cross-section of the plasma channel also enable a plasma-generating device with improved accuracy compared with prior art devices.

It has surprisingly been found that the properties of the plasma-generating device can be affected by variations of the shape of the cathode tip and its position relative to an insulator element arranged along and around the cathode. For example, it has been found that such an insulator element is often damaged due to the high temperature of the cathode tip in the case that the entire cathode tip is positioned inside the insulator element. It has also been found that in operation a spark may occur between the cathode and the insulator element in the case that the entire cathode tip is positioned outside an end face, closest to the anode, of the insulating element, in which case such a spark can damage the insulator element.

According to the invention, the plasma-generating device is provided with an insulator element which extends along and around parts of the cathode, a partial length of said cathode tip projecting beyond a boundary surface of said insulator element. The boundary surface of the insulator element suitably consists of an end face positioned closest to the anode. The insulator element intends to protect parts of the plasma-generating device which are arranged in the vicinity of the cathode from the high temperature thereof in operation. The insulator element is suitably formed as an elongate sleeve with a through hole.

For the proper operation of the plasma-generating device, it is essential that a spark generated at the cathode tip reaches a point in the plasma channel. This is accomplished by positioning the cathode so that the distance between (i) the end of the cathode tip closest to the anode and (ii) the end of the plasma channel closest to the cathode ("cathode end of the plasma channel") is less than or equal to the distance between (a) the end of the cathode tip closest to the anode and (b) any other surface. Preferably, the end of the cathode tip closest to the anode is closer to the cathode end of the plasma channel than to any other point on the surface of the plasma chamber or the insulator element.

By arranging the cathode in such a manner that the tapering tip projects beyond the boundary surface of the insulator element, a distance in the radial direction can be established between the cathode tip and the insulator element portion next to the boundary surface. Such a distance allows a reduction of the risk that the insulator element is damaged by the cathode tip which is hot in operation. Thus, owing to the tapering shape of the cathode tip, the distance between the cathode and the insulator element decreases gradually while the temperature of the cathode decreases away from the hottest tip at the end closest to the anode. An advantage that can be achieved by such an arrangement of the cathode is that the difference in cross-section between the cathode at the base of the cathode tip and the inner dimension of the insulator element can be kept relatively small. Consequently, the outer dimensions of the plasma-generating device can be arranged in a desirable manner for, for instance, keyhole surgery and other space-limited applications.

In an alternative embodiment, substantially half the length of the cathode tip projects beyond said boundary surface of the insulator element. Such a relationship between the position of the cathode tip and the insulator element has been found particularly advantageous to reduce the risk that the insulator element is damaged in operation and to reduce the risk that a spark occurs between the cathode and the insulator sleeve when generating an electric arc between the cathode and the anode.

During operation, a spark may be generated from an edge of the cathode at the base of the cathode tip, which is located at the end of the cathode tip furthest from the anode as well as from the end of the cathode tip closest to the anode. To prevent spark generation from the base of the cathode tip, the cathode is preferably positioned in a way that the end of the cathode tip closest to the anode is closer to the cathode end of the plasma channel than the edge at the base of the cathode tip is to the boundary surface of the insulator element.

In yet another alternative embodiment, the cathode tip of the cathode projects beyond said boundary surface of the insulator element with a length substantially corresponding to a diameter of the base of the cathode tip.

By the length of the cathode tip is meant the length of a tapering part of the cathode end which is directed to the anode. The tapering cathode tip suitably passes into a partial portion of the cathode which has a substantially uniform diameter. In one embodiment, the tapering cathode tip of the cathode is conical in shape. The cathode tip can have, for instance, the shape of a whole cone or a part of a cone. Moreover the base of the cathode tip is defined as a cross-sectional surface, transversely to the longitudinal direction of the cathode, in the position where the cathode tip passes into the partial portion of the cathode with a substantially uniform diameter.

A plasma-generating gas conveniently flows, in operation, between said insulator element and said cathode.

In one embodiment, along a directionally common cross-section through a plane along the base of the cathode tip, a difference in cross-section between a channel arranged in the insulator element and the cathode is equal to or greater than a minimum cross-sectional surface of the plasma channel. The minimum cross-sectional surface of the plasma channel can be positioned anywhere along the extent of the plasma channel. By arranging such a relationship between the cross-sectional surfaces of the cathode and the insulator element, it can be avoided that the space between the cathode and the insulator element constitutes a substantially surge-limiting portion of the flow path of the plasma-generating gas when starting the plasma-generating device. Consequently, this allows that the operating pressure of the plasma-generating device can be established relatively quickly, which allows short start times. Short start times are particularly convenient in the cases that the operator starts and stops the plasma-generating device several times during one and the same use sequence. In one embodiment, the cross-sectional surface of the channel arranged in the insulator element suitably is between 1.5 and 2.5 times the cross-sectional surface of the cathode in a common cross-sectional plane.

In one embodiment of the invention, the insulator element has an inner diameter between 0.35mm and 0.80 mm in the vicinity of the base of the cathode tip, preferably between 0.50 mm and 0.60 mm. However, it will be appreciated that the inner diameter of the insulator element is greater than the diameter of the cathode with a common cross-section, thus forming a space between the two.

The cathode tip of the cathode suitably has a length, which is greater than a diameter of the base of the cathode tip. In one embodiment, the length is equal to or greater than 1.5 times a diameter of the base of the cathode tip. By forming the cathode tip with such a relationship between base diameter and length, it has been found that the shape of the cathode tip provides the possibility of establishing a distance between the cathode tip and the insulator sleeve which is suitable to prevent damage to the insulator sleeve in operation of the plasma-generating device. In an alternative embodiment, the length of the cathode tip is 2-3 times a diameter of the base of the cathode tip.

As mentioned above, at least one embodiment of the plasma-generating device is provided with an insulator element which extends along and around parts of the cathode. The plasma chamber suitably extends between a boundary surface of said insulator element and said opening at the cathode end of the plasma channel. Thus the plasma chamber, or the portion of the plasma chamber where the main part of the plasma is generated, suitably extends from the position where the cathode tip projects beyond the insulator element and up to the opening at the cathode end of the plasma channel.

In one embodiment, a portion tapering towards the anode connects the plasma chamber and the plasma channel. This tapering portion suitably bridges the difference between the cross-section of the plasma chamber and the cross-section of the plasma channel towards the anode. Such a tapering portion allows favourable heat extraction for cooling of structures adjacent to the plasma chamber and the plasma channel.

It has been found convenient to form the cross-sectional surface of the plasma chamber, transversely to the longitudinal direction of the plasma channel, about 4-16 times greater than the cross-sectional surface of the plasma channel. Suitably the cross-sectional surface of the plasma chamber is 4-16 times greater than the cross-sectional surface of the opening of the cathode end of the plasma channel. This relationship between the cross-section of the plasma chamber and that of the plasma channel provides an advantageous space around the cathode tip which reduces the risk that the plasma-generating device is damaged due to high temperatures that may occur in operation.

Preferably, the cross-section of the plasma chamber, transversely to the longitudinal direction of the plasma channel, is circular. It has been found advantageous to form the plasma chamber with a diameter, transversely to the longitudinal direction of the plasma channel, which substantially corresponds to the length of the plasma chamber, in the longitudinal direction of the plasma channel. This relationship between diameter and length of the plasma chamber has been found favourable to reduce the risk of damage due to, for instance, high temperatures that may arise in operation while at the same time reducing the risk that an incorrect electric arc is generated.

It has further been found advantageous to form the plasma chamber with a diameter of the cross-sectional surface of the plasma chamber which corresponds to 2-2.5 times a diameter of the base of the cathode tip.

Suitably also the length of the plasma chamber corresponds to 2-2.5 times a diameter of the base of the cathode tip.

It has surprisingly been found that the properties of the plasma-generating device can be affected by variations of the position of the cathode tip in relation to the opening of the cathode end of the plasma channel. Inter alia, it has been found that the electric arc which is desired to be generated between the cathode and anode when starting the plasma-generating device can be affected. For instance it has been found that an electric arc in an unfavourable manner can occur between the cathode and parts, adjacent to the same, of the plasma-generating device in the case that the cathode tip is positioned too far away from the opening of the cathode end of the plasma channel. Moreover, it has been found that the high temperature of the cathode tip in operation can damage and degrade the plasma channel and/ or material adjoining the same if the cathode tip is positioned too close to the opening at the cathode end of the plasma channel. In one embodiment, it has been found convenient that said cathode tip extends over half the length, or more than half the length, of said plasma chamber. In an alternative embodiment, it has been found suitable to arrange the cathode tip so as to extend over 1/2 to 2/3 of the length of the plasma chamber. In another alternative embodiment, the cathode tip extends over approximately half the length of the plasma chamber.

In one embodiment of the plasma-generating device, the cathode end closest to the anode is positioned at a distance from the opening of the cathode end of the plasma channel, which distance substantially corresponds to the length of that part of the cathode tip which projects beyond the boundary surface of the insulator element.

Moreover, in one embodiment it has been found convenient to arrange the cathode end directed to the anode so that the end of the cathode is positioned at a distance, in the longitudinal direction of the plasma channel, substantially corresponding to a diameter of the base of the cathode tip from the plasma chamber end which is positioned closest to the anode.

By arranging the position of the cathode tip at this distance from the boundary or end of the plasma chamber, in the longitudinal direction of the plasma channel, it has been found that an electric arc can be safely generated while at the same time reducing the risk that material adjoining the plasma channel is damaged by high temperatures in operation.

The plasma chamber is suitably formed by an intermediate electrode positioned closest to the cathode tip. By integrating the plasma chamber as part of an intermediate electrode, a simple construction is provided. Similarly, it is convenient that the plasma channel is formed at least partly by at least one intermediate electrode which is positioned at least partly between said cathode and said anode.

In one embodiment of the plasma-generating device, the plasma chamber and at least parts of the plasma channel are formed by an intermediate electrode which is arranged closest to the cathode tip. In another embodiment the plasma chamber is formed by an intermediate electrode, which is electrically insulated from the intermediate electrodes that form the plasma channel.

As an example of an embodiment of the plasma-generating device, the plasma channel has a diameter which is about 0.20 to 0.50 mm, preferably 0.30-0.40 mm.

In one embodiment, the plasma-generating device comprises two or more intermediate electrodes arranged between said cathode and said anode for forming at least part of the plasma channel. According to an example of an embodiment of the plasma-generating device, the intermediate electrodes jointly form a part of the plasma channel with a length of about 4 to 10 times a diameter of the plasma channel. That part of the plasma channel which extends through the anode suitably has a length of 3-4 times the diameter of the plasma channel. Moreover, an insulator means is suitably arranged between each intermediate electrode and the next. The intermediate electrodes are preferably made of copper or alloys containing copper.

As an example of another embodiment, a diameter of said cathode is between 0.30 and 0.60 mm, preferably 0.40 to 0.50 mm.

According to a second aspect of the invention, a plasma surgical device comprising a plasma-generating device as described above is provided. Such a plasma surgical device of the type here described can suitably be used for destruction or coagulation of biological tissue. Moreover, such a plasma surgical device can advantageously be used in heart or brain surgery. Alternatively, such a plasma surgical device can advantageously be used in liver, spleen or kidney surgery.

### BRIEF DESCRIPTION OF THE DRAWING

The invention will now be described in more detail with reference to the accompanying schematic drawing which by way of example illustrates currently preferred embodiments of the invention.

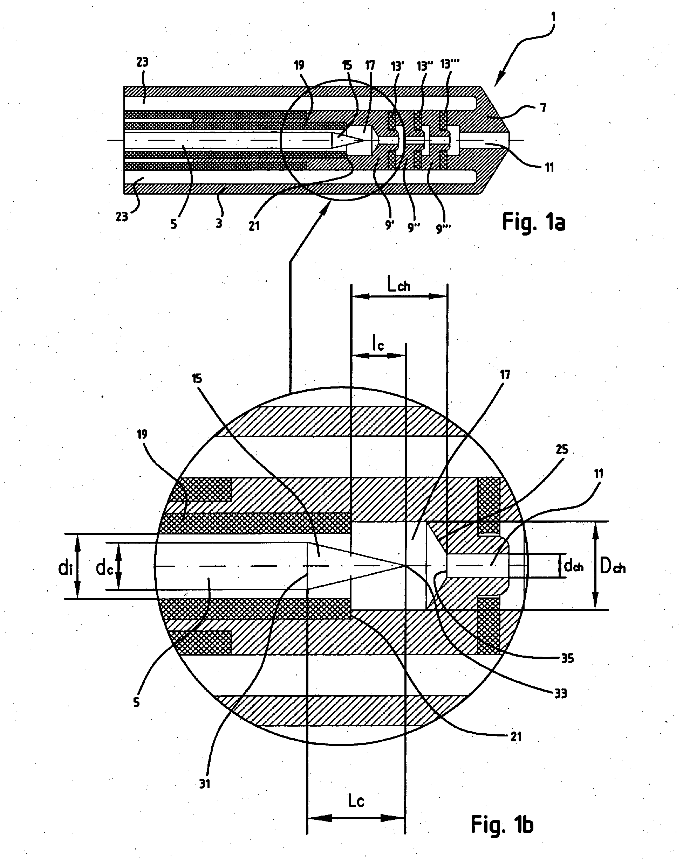

Fig. 1a is a cross-sectional view of an embodiment of a plasma-generating device according to the invention; and
Fig. 1b is a partial enlargement of the embodiment according to Fig. 1a.

### DESCRIPTION OF PREFERRED EMBODIMENTS

Fig. 1a shows in cross-section an embodiment of a plasma-generating device 1 according to the invention. The cross-section in Fig. 1a is taken through the centre of the plasma-generating device 1 in its longitudinal direction. The device comprises an elongate end sleeve 3 which accommodates a plasma-generating system for gene-rating plasma which is discharged at the end of the end sleeve 3. The generated plasma can be used, for instance, to stop bleeding in tissues, vaporise tissues, cut tissues etc.

The plasma-generating device 1 according to Fig. 1a comprises a cathode 5, an anode 7 and a number of electrodes 9', 9", 9'" arranged between the anode and the cathode, in this text referred to as intermediate electrodes. The intermediate electrodes 9', 9", 9'" are annular and form part of a plasma channel 11 which extends from a position in front of the cathode 5 and further towards and through the anode 7. The inlet end of the plasma channel 11 is positioned at the cathode end of the plasma channel. The plasma channel 11 extends through the anode 7 where its outlet end is arranged. In the plasma channel 11, a passing plasma is intended to be heated and finally flow out at the end thereof in the anode 7. The intermediate electrodes 9', 9", 9'" are insulated and separated from direct contact with each other by an annular insulator means 13', 13", 13"'. The shape of the intermediate electrodes 9', 9", 9'" and the dimensions of the plasma channel 11 can be adjusted to any desired purpose. The number of intermediate electrodes 9', 9", 9'" can also be varied in an optional manner. The embodiment shown in Fig. 1a is provided with three intermediate electrodes 9', 9", 9"'.

In the embodiment shown in Fig. 1a, the cathode 5 is formed as an elongate cylindrical element. Preferably, the cathode 5 is made of tungsten, optionally with additives, such as lanthanum. Such additives can be used, for instance, to lower the temperature occurring at the end of the cathode 5.

Moreover the end of the cathode 5 which is directed towards the anode 7 has a tapering end portion 15. This tapering portion 15 suitably forms a tip positioned at the end of the cathode as shown in Fig. 1a. The cathode tip 15 is suitably conical in shape. The cathode tip 15 can also consist of a part of a cone or have alternative shapes with a geometry tapering towards the anode 7.

The other end of the cathode 5 directed away from the anode 7 is connected to an electrical conductor to be connected to an electric energy source. The conductor is suitably surrounded by an insulator. (The conductor is not shown in Fig. 1).

A plasma chamber 17 is connected to the inlet end of the plasma channel 11 and has a cross-sectional surface, transversely to the longitudinal direction of the plasma channel 11, which exceeds the cross-sectional surface of the plasma channel 11 at the inlet end thereof.

The plasma chamber 17 as shown in Fig. 1a is circular in cross-section, transversely to the longitudinal direction of the plasma channel 11, and has an extent in the longitudinal direction of the plasma channel 11 which corresponds approximately to the diameter of the plasma chamber 17. The plasma chamber 17 and the plasma channel 11 are substantially concentrically arranged relative to each other. The cathode 5 extends into the plasma chamber 17 over approximately half the length thereof and the cathode 5 is arranged substantially concentrically with the plasma chamber 17. The plasma chamber 17 consists of a recess integrated in the first intermediate electrode 9', which is positioned next to the cathode 5.

Fig. 1a also shows an insulator element 19 which extends along and around parts of the cathode 5. The insulator element 19 is suitably formed as an elongate cylindrical sleeve and the cathode 5 is partly positioned in a circular hole extending through the tubular insulator element 19. The cathode 5 is arranged substantially in the centre of the through hole of the insulator element 19. Moreover the inner diameter of the insulator element 19 is slightly greater than the outer diameter of the cathode 5, thus forming a distance between the outer circumferential surface of the cathode 5 and the inner surface of the circular hole of the insulator element 19.

Preferably the insulator element 19 is made of a temperature-resistant material, such as ceramic material, temperature-resistant plastic material or the like. The insulator element 19 intends to protect adjoining parts of the plasma-generating device 1 from high temperatures which can arise, for instance, around the cathode 5, in particular around the tip of the cathode 15.

The insulator element 19 and the cathode 5 are arranged relative to each other so that the end of the cathode 5 directed to the anode 7 projects beyond an end face 21, which is directed to the anode 7, of the insulator element 19. In the embodiment shown in Fig. 1a, approximately half the tapering tip 15 of the cathode 5 extends beyond the end face 21 of the insulator element 19.

A gas supply part (not shown in Fig. 1) is connected to the plasma-generating part. The gas supplied to the plasma-generating device 1 advantageously consists of the same type of gases that are used as plasma-generating gas in prior art instruments, for instance inert gases, such as argon, neon, xenon, helium etc. The plasma-generating gas is allowed to flow through the gas supply part and into the space arranged between the cathode 5 and the insulator element 19. Consequently the plasma-generating gas flows along the cathode 5 inside the insulator element 19 towards the anode 7. As the plasma-generating gas passes the end of the insulator element 19 which is positioned closest to the anode 7, the gas is passed into the plasma chamber 17.

The plasma-generating device 1 according to Fig. 1a further comprises additional channels 23 communicating with the elongate end sleeve 3. The additional channels 23 are suitably formed in one piece with a housing which is connected to the end sleeve 3. The end sleeve 3 and the housing can, for instance, be interconnected by a threaded joint, but also other connecting methods, such as welding, soldering etc, are conceivable. Moreover the additional channels 23 can be made, for instance, by extrusion of the housing or mechanical working of the housing. However, it will be appreciated that the additional channels 23 can also be formed by one or more parts which are separate from the housing and arranged inside the housing.

In one embodiment, the plasma-generating device 1 comprises two additional channels 23, one constituting an inlet channel and the other constituting an outlet channel for a coolant. The inlet channel and the outlet channel communicate with each other to allow the coolant to pass through the end sleeve 3 of the plasma-generating device 1. It is also possible to provide the plasma-generating device 1 with more than two cooling channels, which are used to supply or discharge coolant. Preferably water is used as coolant, although other types of fluids are conceivable. The cooling channels are arranged so that the coolant is supplied to the end sleeve 3 and flows between the intermediate electrodes 9', 9", 9"' and the inner wall of the end sleeve 3. The interior of the end sleeve 3 constitutes the area that connects the at least two additional channels to each other.

The intermediate electrodes 9', 9", 9'" are arranged inside the end sleeve 3 of the plasma-generating device 1 and are positioned substantially concentrically with the end sleeve 3. The intermediate electrodes 9', 9", 9'" have an outer diameter which in relation to the inner diameter of the sleeve 3 forms an interspace between the outer surface of the intermediate electrodes and the inner wall of the end sleeve 3. It is in this interspace the coolant supplied from the additional channels 23 is allowed to flow between the intermediate electrodes 9', 9", 9'" and the end sleeve 3.

The additional channels 23 can be different in number and be given different cross-sections. It is also possible to use all, or some, of the additional channels 23 for other purposes. For example, three additional channels 23 can be arranged, where, for instance, two are used for supply and discharge of coolant and one for sucking liquids, or the like, from an area of surgery etc.

In the embodiment shown in Fig. 1a, three intermediate electrodes 9', 9", 9"' are spaced apart by insulator means 13', 13", 13'" which are arranged between the cathode 5 and the anode 7. The first intermediate electrode 9', the first insulator 13' and the second intermediate electrode 9" are press-fitted to each other. Similarly, the second intermediate electrode 9", the second insulator 13" and the third intermediate electrode 9'" are press-fitted to each other. However, it will be appreciated that the number of electrodes 9', 9", 9'" can be selected according to option.

The electrode 9'" which is positioned furthest away from the cathode 5 is in contact with an annular insulator means 13"' which in turn is arranged against the anode 7.

The anode 7 is connected to the elongate end sleeve 3. In the embodiment shown in Fig. 1a, the anode 7 and the end sleeve 3 are formed integrally with each other. In alternative embodiments, the anode 7 can be formed as a separate element which is joined to the end sleeve 3 by a threaded joint between the anode 7 and the end sleeve 3, by welding or by soldering. The connection between the anode 7 and the end sleeve 3 is suitably such as to provide electrical contact between them.

With reference to Fig. 1b suitable geometric relationships between the parts included in the plasma-generating device 1 will be described below. It will be noted that the dimensions stated below merely constitute exemplary embodiments of the plasma-generating device 1 and can be varied according to the field of application and the desired properties.

The inner diameter dᵢ of the insulator element 19 is only slightly greater than the outer diameter d_{c} of the cathode 5. In the embodiment shown in Fig. 1b, the outer diameter d_{c} of the cathode 5 is about 0.50 mm and the inner diameter dᵢ of the insulator element 19 about 0.80 mm.

According to Fig. 1b, the tip 15 of the cathode 5 is positioned in such a manner that about half the length L_{c} of the tip 15 projects beyond a boundary surface 21 of the insulator element 19. In the embodiment shown in Fig. 1b, this projection l_{c} corresponds approximately to the diameter d_{c} of the cathode 5.

The total length L_{c} of the cathode tip 15 suitably corresponds to about 1.5-3 times the diameter d_{c} of the cathode 5 at the base of the cathode tip 31. In the embodiment shown in Fig. 1b, the length L_{c} of the cathode tip 15 corresponds to about 2 times the diameter d_{c} of the cathode 5 at the base of the cathode tip 31. In one embodiment, the cathode 5 is positioned in such a way that the distance between the end of the cathode tip closest to the anode 33 and the cathode end of the plasma channel 35 is less than or equal to the distance between the end of the cathode tip 33 and any other surface, including any surface of plasma chamber 17 and the boundary surface of the insulator element 21. Furthermore, in one embodiment, the cathode is positioned in a way that the distance between the end of the cathode tip 33 and the cathode end of the plasma channel 35 is less than or equal to the distance between the edge at the base of the cathode tip 31 and the boundary surface of the insulator element 21.

In one embodiment, the diameter d_{c} of the cathode 5 is approximately 0.3-0.6 mm at the base of the cathode tip 31. In the embodiment shown in Fig. 1b, the diameter d_{c} of the cathode 5 is about 0.50 mm at the base of the cathode tip 31. Preferably the cathode 5 has a substantially identical diameter d_{c} between the base of the cathode tip 31 and the end, opposite to the cathode tip 15, of the cathode 5. However, it will be appreciated that it is possible to vary this diameter along the extent of the cathode 5.

In one embodiment, the plasma chamber 17 has a diameter D_{ch} which corresponds to approximately 2-2.5 times the diameter d_{c} of the cathode 5 at the base of the cathode tip 31. In the embodiment shown in Fig. 1b, the plasma chamber 17 has a diameter D_{ch} which corresponds to approximately 2 times the diameter d_{c} of the cathode 5.

The extent of the plasma chamber 17 in the longitudinal direction of the plasma-generating device 1 corresponds to approximately 2-2.5 times the diameter d_{c} of the cathode 5 at the base of the cathode tip 31. In the embodiment shown in Fig. 1b, the length L_{ch} of the plasma chamber 17 corresponds to approximately the diameter D_{ch} of the plasma chamber 17.

In the embodiment shown in Fig. 1b, the cathode 5 extending into the plasma chamber 17 is positioned at a distance from the end of the plasma chamber 17 closest to the anode 7 which corresponds to approximately the diameter d_{c} of the cathode tip 31 at the base thereof.

In the embodiment shown in Fig. 1b, the plasma chamber 17 is in fluid communication with the plasma channel 11. The plasma channel 11 suitably has a diameter d_{ch} which is approximately 0.2-0.5 mm. In the embodiment shown in Fig. 1b, the diameter d_{ch} of the plasma channel 11 is about 0.40 mm. However, it will be appreciated that the diameter d_{ch} of the plasma channel 11 can be varied in different ways along the extent of the plasma channel 11 to provide different desirable properties of the plasma-generating device 1.

Between the plasma chamber 17 and the plasma channel 11 a transition portion 25 of the plasma chamber 17 is arranged, which constitutes a tapering transition, away from the cathode 5 to the anode 7, between the diameter D_{ch} of the plasma chamber 17 and the diameter d_{ch} of the plasma channel 11. The transition portion 25 can be formed in a number of alternative ways. In the embodiment shown in Fig. 1b, the transition portion 25 is formed as a bevelled edge which forms a transition between the inner diameter D_{ch} of the plasma chamber 17 and the inner diameter d_{ch} of the plasma channel 11. However, it should be noted that the plasma chamber 17 and the plasma channel 11 can be arranged in direct contact with each other without a transition portion 25.

The plasma channel 11 is formed of the anode 7 and the intermediate electrodes 9', 9", 9'" arranged between the cathode 5 and anode 7. The length of the plasma channel 11 between the opening of the cathode end of the plasma channel and up to the anode suitably corresponds to about 4-10 times the diameter d_{ch} of the plasma channel 11. In the embodiment shown in Fig. 1a, the length of the plasma channel 11 between the opening of cathode end of the plasma channel and the anode is about 2.8 mm.

That part of the plasma channel which extends through the anode is approximately 3-4 times the diameter d_{ch} of the plasma channel 11. For the embodiment shown in Fig. 1a, that part of the plasma channel which extends through the anode has a length of about 2 mm.

The plasma-generating device 1 can advantageously be provided as a part of a disposable instrument. For instance, a complete device with the plasma-generating device 1, outer shell, tubes, coupling terminals etc. can be sold as a disposable instrument. Alternatively, only the plasma-generating device can be disposable and connected to multiple-use devices.

Other embodiments and variants are feasible. For instance, the number and shape of the intermediate electrodes 9', 9", 9'" can be varied according to which type of plasma-generating gas is used and the desired properties of the generated plasma.

In use, the plasma-generating gas, such as argon, which is supplied through the gas supply part, is supplied to the space between the cathode 5 and the insulator element 19 as described above. The supplied plasma-generating gas is passed on through the plasma chamber 17 and the plasma channel 11 to be discharged through the opening of the plasma channel 11 in the anode 7. Having established the gas supply, a voltage system is switched on, which initiates a discharge process in the plasma channel 11 and ignites an electric arc between the cathode 5 and the anode 7. Before establishing the electric arc, it is convenient to supply coolant to the plasma-generating device 1 through the additional channels 23 as described above. Having established the electric arc, a gas plasma is generated in the plasma chamber 17 and is during heating passed on through the plasma channel 11 towards the opening thereof in the anode 7.

A suitable operating current I for the plasma-generating device 1 according to Figs 1a and 1b is suitably less than 10 ampere, preferably 4-6 ampere. The operating voltage of the plasma-generating device 1 is, inter alia, dependent on the number of intermediate electrodes 9', 9", 9'" and the length thereof. A relatively small diameter d_{ch} of the plasma channel 11 enables relatively low energy consumption and relatively low operating current I when using the plasma-generating device 1.

In the electric arc established between the cathode 5 and the anode 7 a temperature T prevails in the centre thereof along the centre axis of the plasma channel 11 and is proportional to the relationship between the discharge current I and the diameter d_{ch} of the plasma channel 11 (T=K*I/d_{ch}). To provide a high temperature of the plasma, for instance 10000 to 15000 °C, at the outlet of the plasma channel 11 in the anode 7, at a relatively low current level I, the cross-section of the plasma channel 11, and thus the cross-section of the electric arc heating the gas, should be small, for instance 0.2-0.5 mm. With a small cross-section of the electric arc, the electric field strength in the plasma channel 11 has a high value.

## Claims

1. A plasma-generating device (1) comprising:
an anode (7);
a cathode (5), said cathode having a tip (15), said tip being a portion of the cathode closest to the anode and tapering toward the anode, said tip having a base (31) at an end furthest from the anode;
a plasma channel (11), extending longitudinally between said cathode and through said anode, and having an outlet opening at the end furthest from said cathode, a part of said plasma channel being formed by one or more, preferably two or more, intermediate electrodes (9', 9", 9"') electrically insulated from each other and said anode; and
a plasma chamber (17) connected to said plasma channel at the cathode end of said plasma channel,
wherein a part of said cathode tip extends longitudinally along a partial length of the plasma chamber, and
wherein a distance between the end of the cathode tip (33) closest to the anode and the cathode end of said plasma channel (35) is less than or equal to a distance between the end of the cathode tip closest to the anode and any other surface,
**characterized in that** the plasma-generating device further comprises a tubular insulator element (19) which extends along and around a portion of the cathode and has a boundary surface (21) at the end closest to the anode, said cathode tip (15) being positioned such that a partial length (I_{c}) of said cathode tip (15) projects beyond the boundary surface (21) of said tubular insulator element (19).

2. The plasma-generating device of claim 1, in which substantially half of the length (L_{c}) of the cathode tip (15) projects beyond said boundary surface (21) of the insulator element (19).

3. The plasma-generating device of claim 1, in which the cathode tip (15) projects beyond said boundary surface (21) of the insulator element (19) by a length which substantially corresponds to a diameter (d_{c}) of the base (31) of the cathode tip.

4. The plasma-generating device of claim 1, in which, along a directionally mutual cross-section through a plane along the base (31) of the cathode tip, a difference between a cross-section of a channel formed by the insulator element (19) and a cross-section of the cathode (5) is equal to or greater than a minimum cross-sectional surface of the plasma channel.

5. The plasma-generating device of claim 1, wherein a plasma-generating gas, in operation, flows between said insulator element (19) and said cathode (5).

6. The plasma-generating device of claim 3, in which a length (L_{c}) of said cathode tip (15) is greater than, preferably equal or greater than 1.5 times, a diameter (d_{c}) of the base (31) of the cathode tip.

7. The plasma-generating device of claim 1, in which at least a half of the length (L_{c}) of the cathode tip (15) extends into said plasma chamber (17).

8. The plasma-generating device of claim 1, in which said plasma chamber (17) extends between said boundary surface (21) of said insulator element (19) and the cathode end of said plasma channel (35).

9. The plasma-generating device of claim 1, in which a cross-sectional surface of the plasma chamber (17) is greater, preferably 4-16 times greater, than a cross sectional surface of the opening of the plasma channel (11) at the cathode end of said plasma channel (35).

10. The plasma-generating device of claim 1, in which a diameter (D_{ch}) of the plasma chamber (17), transversely to the longitudinal direction of the plasma channel substantially corresponds to a length (L_{ch}) of the plasma chamber, in the longitudinal direction of the plasma channel.

11. The plasma-generating device of claim 1, in which said plasma chamber (17) has a tapering portion (25) connected to said plasma channel (11).

12. The plasma-generating device of claim 1, in which said cathode (5) extends into said plasma chamber (17) with a length (I_{c}) corresponding to a diameter (d_{c}) of the base (31) of the cathode tip (15).

13. The plasma-generating device of claim 1, in which the distance between the end (33) of the cathode closest to the anode and the cathode end of the plasma channel (35) substantially corresponds to a diameter (d_{c}) of the base (31) of the cathode tip (15).

14. The plasma-generating device of claim 1, in which said plasma chamber (17) is formed by one of the one or more intermediate electrodes, said one intermediate electrode being closest to the cathode (5).

15. The plasma-generating device of claim 1, in which said plasma chamber (17) and at least parts of said plasma channel (11) are formed by one of the one or more intermediate electrodes, said one intermediate electrodes being the intermediate electrode closest to the cathode (5).

16. The plasma-generating device of claim 1, in which said plasma chamber (17) is formed by an intermediate electrode electrically insulated from the intermediate electrodes forming the plasma channel (11).

17. A plasma surgical device comprising a plasma-generating device according to claim 1.

## Patentansprüche

1. Plasmaerzeugungseinrichtung (1), welche Folgendes aufweist:
eine Anode (7);
eine Kathode (5), wobei die Kathode eine Spitze (15) aufweist, wobei die Spitze ein zur Anode am dichtesten befindlicher Teil der Kathode ist und konisch zur Anode zuläuft, wobei die Spitze eine Grundfläche (31) an einem von der Anode am weitesten entfernten Ende aufweist;
einen Plasmakanal (11), der sich längs zwischen der Kathode und durch die Anode erstreckt und eine Auslassöffnung am von der Kathode am weitesten entfernten Ende aufweist, wobei ein Teil des Plasmakanals durch eine oder mehrere, bevorzugt zwei oder mehr Zwischenelektroden (9', 9", 9"') gebildet ist, die voneinander und von der Anode elektrisch isoliert sind; und
eine Plasmakammer (17), die am Kathodenende des Plasmakanals mit dem Plasmakanal verbunden ist,
wobei ein Teil der Kathodenspitze sich längs entlang einer Teillänge der Plasmakammer erstreckt, und
wobei ein Abstand zwischen dem zur Anode am dichtesten befindlichen Ende der Kathodenspitze (33) und dem Kathodenende des Plasmakanals (35) kleiner als oder gleich einem Abstand zwischen dem zur Anode am dichtesten befindlichen Ende der Kathodenspitze und irgendeiner anderen Fläche ist,
**dadurch gekennzeichnet, dass** die Plasmaerzeugungseinrichtung ferner ein rohrförmiges Isolationselement (19) aufweist, welches sich entlang und um einen Teil der Kathode erstreckt und eine Grenzfläche (21) am zur Anode am dichtesten befindlichen Ende aufweist, wobei die Kathodenspitze (15) derart positioniert ist, dass eine Teillänge (I_{c}) der Kathodenspitze (15) über die Grenzfläche (21) des rohrförmigen Isolationselements (19) hinausragt.

2. Plasmaerzeugungseinrichtung nach Anspruch 1, wobei im Wesentlichen die halbe Länge (L_{c}) der Kathodenspitze (15) über die Grenzfläche (21) des Isolationselements (19) hinausragt.

3. Plasmaerzeugungseinrichtung nach Anspruch 1, wobei die Kathodenspitze (15) über die Grenzfläche (21) des Isolationselements (19) um eine Länge hinausragt, welche im Wesentlichen einem Durchmesser (d_{c}) der Grundfläche (31) der Kathodenspitze entspricht.

4. Plasmaerzeugungseinrichtung nach Anspruch 1, wobei entlang einem direktional wechselseitigen Querschnitt durch eine Ebene entlang der Grundfläche (31) der Kathodenspitze eine Differenz zwischen einem Querschnitt eines durch das Isolationselement (19) gebildeten Kanals und einem Querschnitt der Kathode (5) gleich oder größer als eine minimale Querschnittsfläche des Plasmakanals ist.

5. Plasmaerzeugungseinrichtung nach Anspruch 1, wobei im Betrieb ein Plasmaerzeugungsgas zwischen dem Isolationselement (19) und der Kathode (5) strömt.

6. Plasmaerzeugungseinrichtung nach Anspruch 3, wobei eine Länge (L_{c}) der Kathodenspitze (15) größer als, bevorzugt gleich oder größer als 1,5-mal einem Durchmesser (d_{c}) der Grundfläche (31) der Kathodenspitze ist.

7. Plasmaerzeugungseinrichtung nach Anspruch 1, wobei sich mindestens eine Hälfte der Länge (L_{c}) der Kathodenspitze (15) in die Plasmakammer (17) erstreckt.

8. Plasmaerzeugungseinrichtung nach Anspruch 1, wobei die Plasmakammer (17) sich zwischen der Grenzfläche (21) des Isolationselements (19) und dem Kathodenende des Plasmakanals (35) erstreckt.

9. Plasmaerzeugungseinrichtung nach Anspruch 1, wobei eine Querschnittsfläche der Plasmakammer (17) größer, bevorzugt 4 - 16-mal größer als eine Querschnittsfläche der Öffnung des Plasmakanals (11) am Kathodenende des Plasmakanals (35) ist.

10. Plasmaerzeugungseinrichtung nach Anspruch 1, wobei ein Durchmesser (D_{ch}) der Plasmakammer (17) quer zur Längsrichtung des Plasmakanals im Wesentlichen einer Länge (L_{ch}) der Plasmakammer in Längsrichtung des Plasmakanals entspricht.

11. Plasmaerzeugungseinrichtung nach Anspruch 1, wobei die Plasmakammer (17) einen mit dem Plasmakanal (11) verbundenen, konisch zulaufenden Teil (25) aufweist.

12. Plasmaerzeugungseinrichtung nach Anspruch 1, wobei die Kathode (5) sich mit einer Länge (I_{c}) entsprechend einem Durchmesser (d_{c}) der Grundfläche (31) der Kathodenspitze (15) in die Plasmakammer (17) erstreckt.

13. Plasmaerzeugungseinrichtung nach Anspruch 1, wobei der Abstand zwischen dem zur Anode am dichtesten befindlichen Ende (33) der Kathode und dem Kathodenende des Plasmakanals (35) im Wesentlichen einem Durchmesser (d_{c}) der Grundfläche (31) der Kathodenspitze (15) entspricht.

14. Plasmaerzeugungseinrichtung nach Anspruch 1, wobei die Plasmakammer (17) durch eine der einen oder mehreren Zwischenelektroden gebildet ist, wobei die eine Zwischenelektrode sich zur Kathode (5) am dichtesten befindet.

15. Plasmaerzeugungseinrichtung nach Anspruch 1, wobei die Plasmakammer (17) und mindestens Teile des Plasmakanals (11) durch eine der einen oder mehreren Zwischenelektroden gebildet sind, wobei die eine Zwischenelektrode die zur Kathode (5) am dichtesten befindliche Zwischenelektrode ist.

16. Plasmaerzeugungseinrichtung nach Anspruch 1, wobei die Plasmakammer (17) durch eine Zwischenelektrode gebildet ist, die von den den Plasmakanal (11) bildenden Zwischenelektroden elektrisch isoliert ist.

17. Plasmachirurgievorrichtung, welche eine Plasmaerzeugungseinrichtung nach Anspruch 1 aufweist.

## Revendications

1. Dispositif de génération de plasma (1) comprenant :
une anode (7) ;
une cathode (5), ladite cathode ayant une pointe (15), ladite pointe étant une partie de la cathode la plus proche de l'anode et s'effilant vers l'anode, ladite pointe ayant une base (31) au niveau d'une extrémité la plus éloignée de l'anode ;
un canal à plasma (11) s'étendant longitudinalement entre ladite cathode et à travers ladite anode, et ayant une ouverture de sortie au niveau de l'extrémité la plus éloignée de ladite cathode, une partie dudit canal à plasma étant formée par une ou plusieurs, de préférence au moins deux, électrodes intermédiaires (9', 9", 9"') électriquement isolées les unes des autres et de ladite anode; et
une chambre à plasma (17) raccordée audit canal à plasma au niveau de l'extrémité de la cathode dudit canal à plasma,
où une partie de ladite pointe de cathode s'étend longitudinalement sur une longueur partielle de la chambre à plasma, et
où une distance entre l'extrémité de la pointe de cathode (33) la plus proche de l'anode et l'extrémité de la cathode dudit canal à plasma (35) est inférieure ou égale à une distance entre l'extrémité de la pointe de cathode la plus proche de l'anode et de toute autre surface,
**caractérisée en ce que** le dispositif de génération de plasma comprend en outre un élément isolant tubulaire (19) qui s'étend le long et autour d'une partie de la cathode et a une surface limite (21) au niveau de l'extrémité la plus proche de l'anode, ladite pointe de cathode (15) étant positionnée de telle sorte qu'une longueur partielle (I_{c}) de ladite pointe de cathode (15) fait saillie au-delà de la surface limite (21) dudit élément isolant tubulaire (19).

2. Dispositif de génération de plasma selon la revendication 1, dans lequel sensiblement la moitié de la longueur (L_{c}) de la pointe de cathode (15) fait saillie au-delà de la surface limite (21) de l'élément isolant (19).

3. Dispositif de génération de plasma selon la revendication 1, dans lequel la pointe de cathode (15) fait saillie au-delà de la surface limite (21) de l'élément isolant (19) par une longueur qui correspond sensiblement au diamètre (d_{c}) de la base (31) de la pointe de cathode.

4. Dispositif de génération de plasma selon la revendication 1, dans lequel, le long d'une section transversale directionnellement mutuelle à travers un plan le long de la base (31) de la pointe de cathode, une différence entre une section transversale d'un canal formé par l'élément isolant (19) et une section transversale de la cathode (5) est égale ou supérieure à une surface de section transversale minimale du canal à plasma.

5. Dispositif de génération de plasma selon la revendication 1, dans lequel un gaz de génération de plasma, pendant le fonctionnement, circule entre ledit élément isolant (19) et ladite cathode (5).

6. Dispositif de génération de plasma selon la revendication 3, dans lequel une longueur (L_{c}) de ladite pointe de cathode (15) est supérieure, de préférence égale ou supérieure à 1,5 fois, à un diamètre (d_{c}) de la base (31) de la pointe de cathode.

7. Dispositif de génération de plasma selon la revendication 1, dans lequel au moins une moitié de la longueur (L_{c}) de la pointe de cathode (15) s'étend dans ladite chambre à plasma (17).

8. Dispositif de génération de plasma selon la revendication 1, dans lequel ladite chambre à plasma (17) s'étend entre ladite surface limite (21) dudit élément isolant (19) et l'extrémité de la cathode dudit canal à plasma (35).

9. Dispositif de génération de plasma selon la revendication 1, dans lequel une surface de section transversale de la chambre à plasma (17) est supérieure, de préférence 4 - 16 fois supérieure, à une surface de section transversale de l'ouverture du canal à plasma (11) à l'extrémité de la cathode dudit canal à plasma (35).

10. Dispositif de génération de plasma selon la revendication 1, dans lequel un diamètre (D_{ch}) de la chambre à plasma (17), transversalement à la direction longitudinale du canal à plasma correspond sensiblement à une longueur (L_{ch}) de la chambre à plasma, dans la direction longitudinale du canal à plasma.

11. Dispositif de génération de plasma selon la revendication 1, dans lequel ladite chambre à plasma (17) a une partie effilée (25) raccordée audit canal à plasma (11).

12. Dispositif de génération de plasma selon la revendication 1, dans lequel ladite cathode (5) s'étend dans ladite chambre à plasma (17) avec une longueur (I_{c}) correspondant à un diamètre (d_{c}) de la base (31) de la pointe de cathode (15).

13. Dispositif de génération de plasma selon la revendication 1, dans lequel la distance entre l'extrémité (33) de la cathode la plus proche de l'anode et l'extrémité de la cathode du canal à plasma (35) correspond sensiblement à un diamètre (d_{c}) de la base (31) de la pointe de cathode (15).

14. Dispositif de génération de plasma selon la revendication 1, dans lequel ladite chambre à plasma (17) est formée par une de la ou des électrodes intermédiaires, ladite une électrode intermédiaire étant la plus proche de la cathode (5).

15. Dispositif de génération de plasma selon la revendication 1, dans lequel ladite chambre à plasma (17) et au moins certaines parties dudit canal à plasma (11) sont formées par une de la ou des électrodes intermédiaires, ladite une électrode intermédiaire étant l'électrode intermédiaire la plus proche de la cathode (5).

16. Dispositif de génération de plasma selon la revendication 1, dans lequel ladite chambre à plasma (17) est formée par une électrode intermédiaire électriquement isolée des électrodes intermédiaires formant le canal à plasma (11).

17. Dispositif chirurgical à plasma comprenant un dispositif de génération de plasma selon la revendication 1.
